# EUROPEAN PATENT SPECIFICATION

(11) **EP 3 385 368 B1**
(45) Date of publication and mention of the grant of the patent: **01.03.2023**
(21) Application number: 16870814.7
(22) Date of filing: 02.12.2016
(51) Int. Cl.: C12N 1/00, A61K 35/28, C12N 5/0775

(54) **METHOD FOR PRODUCING MESENCHYMAL STEM CELLS**
VERFAHREN ZUR HERSTELLUNG MESENCHYMALER STAMMZELLEN
PROCÉDÉ DE PRODUCTION DE CELLULES SOUCHES MÉSENCHYMATEUSES

(30) Priority: 03.12.2015 JP 2015236442
(43) Date of publication of application: 10.10.2018
(73) Proprietor: Takara Bio Inc., Kusatsu-shi Shiga 525-0058 (JP)
(72) Inventor: TAHARA, Kenichi, Kusatsu-shi Shiga 525-0058 (JP); NUKAYA, Ikuei, Kusatsu-shi Shiga 525-0058 (JP); IDENO, Mitsuko, Kusatsu-shi Shiga 525-0058 (JP); YAMAGUCHI, Saori, Kusatsu-shi Shiga 525-0058 (JP); MINENO, Junichi, Kusatsu-shi Shiga 525-0058 (JP)
(74) Representative: Müller, Christian Stefan Gerd
(86) International application number: PCT/JP2016/085882
(87) International publication number: WO 2017/094879

(56) References cited:
- WO-A1-2007/023875
- WO-A1-2008/023771
- JP-A- 2007 000 077
- JP-A- 2007 000 077
- JP-A- 2008 048 653
- MAXIM A VODYANIK ET AL: "A Mesoderm-Derived Precursor for Mesenchymal Stem and Endothelial Cells", CELL STEM CELL, ELSEVIER, CELL PRESS, AMSTERDAM, NL, vol. 7, no. 6, 13 September 2010 (2010-09-13), pages 718-729, XP028211788, ISSN: 1934-5909, DOI: 10.1016/J.STEM.2010.11.011 [retrieved on 2010-11-17]

## Description

### Technical Field

The present invention relates to a production method of a mesenchymal stem cell (MSC), in particular a production method of an adipose-derived stem cell (ASC or ADSC). The present disclosure also describes a mesenchymal stem cell obtained by the method, and a composition for regenerative medicine containing the cell as an effective ingredient.

### Background Art

Mesenchymal stem cells are somatic stem cells derived from mesodermal tissue (mesenchyme). Since mesenchymal stem cells have the ability to differentiate into cells belonging to mesenchyme, they are expected to be applied to regenerative medicine including rebuilding of bone, blood vessel, cardiac muscle and the like. In addition, mesenchymal stem cells are available as research tools in the fields of embryology, molecular biology, and pharmacy. It is known that MSC can be obtained from various tissues including bone marrow, adipose tissue, blood, placentas, umbilical cords, and dental pulps. Since MSC has a different property depending on the tissue from which MSC is obtained, MSC is called a bone marrow-derived stem cell, adipose-derived stem cell or the like depending on the tissue from which MSC is obtained.

Mesenchymal stem cells were conventionally obtained from bone marrow. In bone marrow, however, there are only a small amount of mesenchymal stem cells, and the number of mesenchymal stem cells decreases with age. Thus, from the point of view of clinical application, it is necessary to collect several hundred milliliters of bone marrow in order to obtain enough number of mesenchymal stem cells, which is a great burden to patients. On the other hand, mesenchymal stem cells are abundant in subcutaneous adipose. Since subcutaneous adipose is easily obtained by aspiration of fat under local anesthesia, a burden to patients is small. In recent years, clinical researches using adipose-derived stem cells are increasing.

A general method for obtaining adipose-derived stem cells comprises subjecting a small amount of a fat section taken from human to enzymatic treatment to obtain a cell population, separating a stromal vascular fraction (SVF) that is sedimentary from the cell population by centrifugation, and continuously culturing the fraction in a medium containing a bovine serum to proliferate (Non-Patent Literature 1). However, the use of a bovine serum which is an animal-derived component has a great problem from the point of view of clinical application.

### Citation List

### Non-Patent Literatures

Non-patent literature 1: Tissue Engineering, 2001, Vol.7, No.2, pages 211-218

### Summary of Invention

### Problem to be solved by the Invention

The present invention solves the above-mentioned problems that the conventional production methods of mesenchymal stem cells had. An objective of the present invention is to provide a method of producing a large amount of mesenchymal stem cells in a short time.

### Solutions to the Problems

The inventors of the present invention intensively studied for accomplishing the objective, and as a result, they found that a large amount of mesenchymal stem cells were produced in a xeno-free medium in a short time by culturing a cell population containing a mesenchymal stem cell in the presence of a fibronectin fragment. Thus, the present invention was completed.

The present invention is defined by the appended claims which are directed to the following:
[1] a method of producing a mesenchymal stem cell, the method comprising a step of culturing a cell population containing a mesenchymal stem cell in the presence of a fibronectin fragment using a medium containing plasma, wherein the cell population containing a mesenchymal stem cell is a cell population directly separated from adipose tissue,
[2] the method according to [1], wherein the fibronectin fragment is a fragment having at least one domain selected from the group consisting of a cell-adhesion domain and a heparin-binding domain,
[3] the method according to [1], wherein the culturing step in the presence of a fibronectin fragment is a step of culturing the cell population in contact with a solid phase coated with the fibronectin fragment,
[4] the method according to [1], wherein the culturing step is a step of culturing the cell population in a xeno-free medium,
[5] the method according to [4], wherein the xeno-free medium is a serum-free medium or a medium containing an allogeneic serum,
[6] the method according to [1], wherein the cell population is a cell population derived from a human,
[7] a method of producing a cell belonging to mesenchyme, the method comprising a step of obtaining a mesenchymal stem cell by the method according to any one of [1]-[6] and a step of differentiating the mesenchymal stem cell, and
[8] the method according to [7], wherein the differentiation step is a culturing step in a medium containing a differentiation inducer that induces differentiation of a mesenchymal stem cell into another cell.

### Effects of the Invention

The present invention enables production of a large amount of mesenchymal stem cells in a xeno-free medium in a short time.

### Brief Description of Drawings

[Fig. 1] Figure 1 shows microscopic images on the 8th day after culture was started in Example 1. The left image: condition A, the central image: condition B, the right image: condition C.
[Fig. 2] Figure 2 shows the number of culture days and the number of cells collected in Example 2.
[Fig. 3] Figure 3 shows differentiation of mesenchymal stem cells into various cells in Example 3.

### Mode for Carrying out the Invention

### Definition etc.:

As used herein, the term "mesenchymal stem cells (MSC)" means somatic stem cells derived from mesodermal tissue (mesenchyme).

Mesenchymal stem cells are known to express specific cell surface markers such as CD73, CD90, and CD105. The mesenchymal stem cells present in a sample as the starting material or a cell population finally obtained can be detected or quantified by testing the expression of these markers by an immunologic method (detecting them by using antibodies).

As used herein, the term "adipose-derived stem cells (ADSC or ASC)" means stem cells present in adipose tissue. Adipose-derived stem cells are included in the mesenchymal stem cells, and retain the same differentiation ability as the mesenchymal stem cells.

As used herein, the term "allogenic" means that organisms or genes or the like that the organisms have are derived from the same species as a criterial species. The term "allogenic" includes isogeneic and autologous.

As used herein, the term "heterologous" means that organisms or genes or the like that the organisms have are derived from a species different from a criterial species.

As used herein, the term "xeno-free" means containing no component derived from a species different from a criterial species.

Hereinafter, the present invention is explained in detail.

### (1) Production method of mesenchymal stem cell of the present invention

### <Cell population containing mesenchymal stem cell>

The method of producing a mesenchymal stem cell of the present invention includes a step of culturing a cell population containing a mesenchymal stem cell in the presence of a fibronectin fragment. Hereinafter, the "cell population containing a mesenchymal stem cell" is explained in detail.

The "cell population containing a mesenchymal stem cell" is not particularly limited, and may be any cell population containing a mesenchymal stem cell but has to be a cell population directly separated from adipose tissue.

Organisms from which cells used in the present invention are derived are not particularly limited. The cells used in the present invention may be derived from any organisms, preferably mammals. The ages and sexes of the organisms are not particularly limited. In an embodiment, cells derived from primates (for example, chimpanzees, Macaca fuscata, humans) are used in the present invention. Human cells are used most preferably, but the present invention is not limited to use of human cells. In the case where mesenchymal stem cells are produced by the present invention for the purpose of administration to humans, a cell population obtained from a donor having the same histocompatibility antigen type as or a similar histocompatibility antigen to that of a recipient is preferably used as a material. More preferably, a cell population obtained from a recipient is used for production of mesenchymal stem cells.

The "cell population containing a mesenchymal stem cell" may be primary cells that are directly separated from living tissue containing mesenchymal stem cells. As used herein, the term "directly" means that an ex vivo culture/proliferation step is not performed.

The living tissue containing mesenchymal stem cells is adipose tissue. The adipose tissue can be obtained by aspiration of fat or adipectomy which results in a little risk for dysfunction in individuals, and thus is suitable as a source of the above-mentioned cell population.

The adipose tissue is composed of adipocytes and is a kind of living tissues. Examples of sites from which the adipose tissue used in the present invention is derived include, but not particularly limited to, subcutaneous fat, visceral fat, intramuscular fat, and intermuscular fat. Among them, the subcutaneous fat is a preferable source of the adipose tissue because it can be easily obtained under local anesthesia.

A method of preparing the adipose tissue is not particularly limited. The adipose tissue can be prepared from pieces of tissue aspirated by liposuction in cosmetic surgery or excised adipose tissue contained in tissue excised from a living body in surgery or the like. Since adipose-derived stem cells are present around large blood vessels, they can be obtained in a larger amount from excised adipose tissue than fat aspirate fluid. On the other hand, preparation of the stem cells from fat aspirate fluid leaves a smaller operative scar, which imposes a smaller burden on a donor.

Though one kind of adipose tissue is usually used, two or more kinds of adipose tissues can be also used in combination. The adipose tissue may be collected multiple times, and the collected adipose tissues may be mixed and then used.

The collection amount of adipose tissue can be determined depending on the kind of a donor, the kind of donor's tissue, or the necessary amount of a mesenchymal stem cell. Though the present invention should not be particularly limited, mesenchymal stem cells can be obtained from 0.3 to 20 g of adipose tissue. A larger amount of adipose tissue can be used as the starting material by using scale-up or multiple operations.

The collected adipose tissue is subjected to removal of blood components attached to it and chopping into small pieces, if needed. Then, the collected adipose tissue is subjected to an enzymatic treatment (protease treatment) as described below. The blood components can be removed by washing the adipose tissue with a suitable buffer or a suitable culture medium.

The enzymatic treatment is performed by digesting the adipose tissue with protease such as collagenase, trypsin, Dispase or the like. Such an enzymatic treatment may be performed according to methods and conditions known to a person skilled in the art (see, for example, R. I. Freshney, Culture of Animal Cells: A Manual of Basic Technique, 4th Edition, A John Wiley & Sones Inc., Publication). Preferably, the enzymatic treatment is performed according to the methods and conditions as described later in Examples.

The enzymatically-treated adipose tissue contains two major cell populations: a stromal vascular fraction and mature adipocytes. The stromal vascular fraction is a cell mixture consisting of preadipocytes, mature endothelial cells, endothelial progenitor cells, vascular smooth muscle cells, pericytes, parietal cells, macrophages, fibroblasts, and adipose-derived stem cells. The adipose-derived stem cell is a mesenchymal stem cell capable of easily differentiating into an adipocyte, an osteoblast, a chondrocyte or the like. The kinds, ratios and the like of cells constituting the cell population depend on the origin and kind of the used adipose tissue.

The enzymatically-treated adipose tissue is then centrifuged to separate the two cell populations from each other. After the centrifugation, a precipitate is collected as a precipitated cell population (comprising the stromal vascular fraction). Conditions for the centrifugation vary on the kinds and amounts of cells. For example, the centrifugation is performed at 300 to 2000 x g for 1 to 15 minutes. Before centrifugation, the enzymatically-treated cell population may be subjected to filtration or the like to remove enzymatically-undigested tissue and the like. For the filtration, for example, a filter having pore size of 50-200 um, preferably 100 um may be used. In the present invention, the stromal vascular fraction can be used as the "cell population containing a mesenchymal stem cell".

### <Fibronectin fragment>

Fibronectin is a huge glycoprotein having a molecular weight of 250,000 present in blood, on a cultured cell surface, and in an extracellular matrix of a tissue, of animals, and is known to have various functions. The domain structure of fibronectin is divided into seven. The amino acid sequence of the domain structure includes three kinds of similar sequences, and the whole is comprised of repeats of these sequences. The three kinds of similar sequences are called type I, type II and type III. Type III is comprised of 71 to 96 amino acid residues, and identity among these amino acid residues is 17 to 40%. There are 14 type III sequences in fibronectin and, among them, the eighth, ninth and tenth sequences (hereinafter, referred to as III-8, III-9 and III-10, respectively) are contained in a cell-adhesion domain, and the twelfth, thirteenth and fourteenth sequences (hereinafter, referred to as III-12, III-13 and III-14, respectively) are contained in a heparin-binding domain. III-10 contains a region having a binding activity to integrin α₅β₁ (referred to as VLA-5), and the core sequence thereof is RGD. In addition, a region called IIICS exists at the C-terminal side of fibronectin. In IIICS, there is a sequence called CS-1 which consists of 25 amino acid residues, and the sequence exhibits a binding activity to integrin α₄β₁ (referred to as VLA-4).

The amino acid sequences of III-8 to III-14 and CS-1 are shown as SEQ ID NOs: 1 to 7 and 8 in a sequence listing which is a part of the specification. In the present invention, any fibronectin fragment may be used as long as it has a feature suitable to culture of a mesenchymal stem cell. For example, a fibronectin fragment containing one or more amino acid sequences selected from the above-mentioned amino acid sequences can be used.

As fibronectin fragments, many fragments containing a cell adhesion domain or a heparin-binding domain within the molecules are known (see, for example, J. Biochem., vol. 110, pp. 284-291, 1991).

Examples of the fibronectin fragment used in the present invention include, but not particularly limited to, fragments containing at least one domain selected from the group consisting of a cell adhesion domain (III-8, III-9, and III-10) and a heparin-binding domain (III-12, III-13, and III-14).

As used herein, the fibronectin fragment may be a fragment including a functional domain of fibronectin as described above, that is, the cell adhesion domain or the heparin-binding domain, and does not have to consist of a part of a continuous amino acid sequence of fibronectin. The fibronectin fragment can be produced by fragmentation of naturally-occurring fibronectin by enzymatic digestion, or by recombinant DNA technology. Recombinant fibronectin fragments are suitable in the present invention, in consideration of less effort required for removal of impurities and designing and obtaining a desired fragment regardless of an enzymatic recognition site or the like. In terms of the production or handling of recombinants, it is preferred that the fibronectin fragments used in the present invention have a molecular weight of not more than 100 kDa.

An example of a fibronectin fragment having both the cell adhesion domain and the heparin-binding domain is RetroNectin (registered trademark, manufactured by TAKARA BIO INC.). RetroNectin is described as CH-296 in J. Biochem. forecited. RetroNectin is a recombinant protein having a molecular weight of about 63000 (574 amino acid residues), comprising the cell adhesion domain (III-8, III-9, and III-10), the heparin-binding domain (III-12, III-13, and III-14), and CS-1. The amino acid sequence of RetroNectin is shown as SEQ ID NO:9 in the sequence listing which is a part of the specification.

As used herein, the fibronectin fragments include proteins chemically modified by acetylation and the like. For the production of a mesenchymal stem cell in the present invention, single molecule species of a fibronectin fragment or a mixture of two or more molecule species of fibronectin fragments may be used.

Substances functionally equivalent to the fibronectin fragments, for example, substances having a cell adhesion domain and/or a heparin-binding domain can be also used. Derivatives of the fibronectin fragments can be also used.

The culture in the presence of the fibronectin fragment of the present invention may be performed by any method wherein the cell population containing a mesenchymal stem cell is contacted with the fibronectin fragment. For example, a medium dissolving the fibronectin fragment may be used for the culture, or a solid phase on which the fibronectin fragment is immobilized may be used for the culture. A preferable aspect of the present invention includes a step of culturing the cell population containing a mesenchymal stem cell in contact with a solid phase coated with the fibronectin fragment. Hereinafter, the "solid phase coated with the fibronectin fragment" will be explained in detail.

In a preferable aspect of the present invention, the fibronectin fragment is used in a state of coating a suitable solid phase, for example a vessel or a carrier (microbeads etc.) used for cell culture. A culture vessel which is used may be of any material and in any shape as long as the material or the shape does not inhibit maintenance, survival, differentiation, maturation, and self-renewal of cells. Examples of the material of the culture vessel include glass, synthetic resin and natural resin including unwoven fabric, and metals. Examples of the shape of the culture vessel include a polygonal column such as a triangle pole, a cube and a cuboid, a cylinder, a polygonal pyramid such as a triangular pyramid and a quadrangular pyramid, a circular cone, any shape like a gourd, a globular shape, a hemispherical shape, a round shape, a shape of an ellipse, and a semicircular shape. A commercially available culture flask, culture dish, culture bag, hollow fiber culture instrument or the like can be also used.

The culture bag is preferably a gas-permeable culture bag. In the case where a large amount of cells are needed, a large culture tank may be used. Although cell culture can be performed in either an open system or a closed system, it is preferable that the cell culture is performed in a closed system for the purpose of administering the obtained mesenchymal stem cells to human.

Coating of the solid phase surface with the fibronectin fragment may be performed by a known method. For example, a solution of the fibronectin fragment in sterile water, a buffer, saline or the like can be used for the coating. The fibronectin fragment is preferably dissolved in phosphate buffered saline (PBS).

The amount of the fibronectin fragment used in the present invention is not particularly limited, and can be determined by a person skilled in the art. For example, when RetroNectin is used as the fibronectin fragment, the coating can be performed by adding PBS containing RetroNectin at a final concentration of 20 ug/mL into a culture vessel and incubating the vessel at room temperature for an hour.

The vessel coated with the fibronectin fragment can be stored at a low temperature, for example 4°C until use. Just before use, the fibronectin fragment-containing solution is removed from the culture instrument by suction. The culture instrument is washed with PBS one time and then each cell culture medium one time, and then provided for the cell culture.

### <Culture>

In the method of producing a mesenchymal stem cell of the present invention, the "step of culturing a cell population containing a mesenchymal stem cell in the presence of a fibronectin fragment" comprises a step of isolating and culturing a mesenchymal stem cell, or a step of expansion of a mesenchymal stem cell, or both a step of isolating and culturing a mesenchymal stem cell and a step of expansion of a mesenchymal stem cell. Thus, the method of producing a mesenchymal stem cell of the present invention may be a method of isolating and culturing a mesenchymal stem cell which includes the "step of culturing a cell population containing a mesenchymal stem cell in the presence of a fibronectin fragment", or an expansion method of a mesenchymal stem cell which includes the "step of culturing a cell population containing a mesenchymal stem cell in the presence of a fibronectin fragment". A preferable example of the present invention is a method of obtaining a mesenchymal stem cell by two steps of the isolation and culture of the mesenchymal stem cell and the expansion of the isolated and cultured mesenchymal stem cell. Hereinafter, these steps will be explained in detail.

### (i) Isolation and culture of mesenchymal stem cell

In this step, the desired mesenchymal stem cell is selectively proliferated by culturing a cell population containing a mesenchymal stem cell in the presence of a fibronectin fragment.

A medium for this step can be prepared based on a medium usually used for animal cell culture. A preferable example of the medium includes a medium that does not contain a heterologous component such as a fetal bovine serum (FBS) or a fetal calf serum (FCS), or a sheep serum. Although such a xeno-free medium can be prepared as appropriate, a known medium or a commercially available medium may be used as it is or after alteration. Examples of the commercially available xeno-free medium include DEF-CS500 XF (manufactured by Cellartis), and DXF (manufactured by PromoCell).

In the case where a mesenchymal stem cell is produced from an autologous tissue, the medium may be a medium containing an allogeneic serum or a serum-free medium. Preferable examples of such a medium include a medium containing an allogeneic serum and containing no heterologous component, and a serum-free medium containing no heterologous component. The allogeneic serum is preferably an autologous serum. As used herein, the autologous serum, and autologous plasma as mentioned later mean a serum and plasma obtained from blood taken from the same donor as that for a cell population to be cultured.

Since plasma contains serum components, a medium containing plasma is used in the methods of the present invention. Preferably, inactivated autologous plasma is added to a medium. For example, the cells are cultured in a medium containing 10%(V/V) or less, preferably 5%(V/V) or less, more preferably 2%(V/V) or less of inactivated autologous plasma. The use of autologous plasma allows exclusion of heterologous components from the production steps, and thereby, a safe cell production method is provided.

Conditions for the cell culture are not particularly limited, and normal cell culture conditions can be used. Examples of the cell culture conditions include, but not limited to, cell culture under at a temperature of 37°C, a humidity of 95% and a CO₂ concentration of 5%. For example, although the cell culture may be performed under at a temperature of 30-40°C, a humidity of 90-98% and a CO₂ concentration of 3-7%, the temperature, humidity and CO₂ concentration do not have to fall within the above-mentioned ranges as long as the desired cell proliferation can be attained. It is preferable that a medium containing effective ingredients is exchanged for the fresh medium at a suitable time interval during culture.

In a preferable aspect of the present invention, the stromal vascular faction is cultured in a xeno-free medium to which 5% autologous plasma has been added in a vessel coated with a fibronectin fragment, for example, for 4-14 days, preferably 7-10 days, while the medium is exchanged for fresh one every 3 or 4 days. This culture results in selective proliferation of a mesenchymal stem cell. In other words, mesenchymal stem cells occupy 80% or more, preferably 90% or more of the cell population obtained after the cell culture.

The cell concentration at the start of culture in the isolation and culture step is not particularly limited, and examples thereof include 0.1 to 10 × 10⁵ cells/mL, preferably 0.3 to 5 × 10⁵ cells/mL, and more preferably 0.5 to 2 × 10⁵ cells/mL.

### (ii) Expansion of mesenchymal stem cell

In this step, a mesenchymal stem cell is subjected to expansion culture in the presence of a fibronectin fragment to allow the mesenchymal stem cell to proliferate massively. The mesenchymal stem cell to be subjected to the culture in this step is the mesenchymal stem cell isolated by the above-mentioned step (i).

The mesenchymal stem cell isolated by the above-mentioned step (i) may be separated from the culture vessel by a method usually used in the art, for example, a physical method, a method comprising use of a chelating agent, an enzymatic method comprising use of a separating solution having protease activity and/or collagenase activity (for example, Accutase (registered trademark) and Accumax (registered trademark) etc.), or any combination of them. A preferable method comprises enzymatically dissociating mesenchymal stem cells from a sheet and then physically finely dispersing the cells. The cell to be used is preferably a cell cultured to 70-95% confluency in the used culture vessel, more preferably a cell cultured to 80-90% confluency in the used culture vessel.

The separated cell population can be used as it is or after freeze storage. A subset of cells that are separated based on a specific cell surface marker from the above-mentioned cell population, for example an isolated mesenchymal stem cell can be also used in this step.

A medium and culture conditions for this step are the same as described for the above-mentioned step (i). This step may be performed using the same medium, vessel and culture conditions as those used in the above-mentioned step (i) or using a medium, vessel and culture conditions different from those used in the above-mentioned step (i). In the same manner as the culture in the above-mentioned step (i), a medium that does not contain a heterologous component is preferably used, and any xeno-free medium, for example a known medium or a commercially available medium may be used as it is of after alteration.

In a preferable aspect of the present invention, the isolated and cultured mesenchymal stem cell is expanded in a xeno-free medium to which a serum has not been added in a vessel coated with a fibronectin fragment, for example, for 4-14 days, preferably 6-12 days, while the medium is exchanged for fresh one every 3 or 4 days and the cell is subcultured as appropriate. This culture results in massive proliferation of the mesenchymal stem cell. In other words, the mesenchymal stem cell is proliferated up to 100 times or more, preferably 300 times or more by this culture.

The cell concentration at the start of culture in the expansion step is not particularly limited, and examples thereof include 0.1 to 10 × 10⁵ cells/mL, preferably 0.3 to 5 × 10⁵ cells/mL, and more preferably 0.5 to 2 × 10⁵ cells/mL.

### (2) Mesenchymal stem cell obtained by the method of the present invention

A mesenchymal stem cell or a cell population containing a mesenchymal stem cell can be obtained by the method of producing a mesenchymal stem cell of the present invention as described above.

The mesenchymal stem cell obtained by the method of the present invention can be confirmed by detecting a molecule which is characteristic of a mesenchymal stem cell, such as an enzyme, a receptor, a low-molecular weight compound or the like. Examples of the molecule which is characteristic of a mesenchymal stem cell include, but not limited to, CD73, CD90, CD105, CD166 and the like which are cell surface makers (positive markers). CD19, CD34, CD45, HLA-DR, CD11b, CD14 and the like are also known as negative markers which are not expressed on a mesenchymal stem cell, and they can be used for confirmation of a mesenchymal stem cell. The above-mentioned molecules may be detected by immunological methods, or by quantifying the mRNA amount of each molecule.

The above-mentioned positive markers are expressed on for example 80% or more, preferably 90% or more, further preferably 95% or more of the cell population containing a mesenchymal stem cell obtained by the method of the present invention. An expression rate of the negative markers in the cell population containing a mesenchymal stem cell obtained by the method of the present invention is for example 5% or less, preferably 1% or less, further preferably less than a detection limit.

An antibody that recognizes the molecule which is characteristic of a mesenchymal stem cell is also useful for isolation and purification of the mesenchymal stem cell obtained by the method of the present invention. Disclosed herein is a cell population obtained by isolating, separating and purifying mesenchymal stem cells based on the molecule which is characteristic of a mesenchymal stem cell.

The mesenchymal stem cell obtained by the method of the present invention can be also used, for example, in research concerning differentiation of mesenchymal stem cells, screening of drugs for various diseases, evaluation of efficacy and safety of drug candidate compounds, and the like. According to the present invention, a lot of mesenchymal stem cells can be obtained by one stage, and therefore, reproducible experimental results can be obtained without being affected by differences between lots of the cells.

### (3) Therapeutic composition

A therapeutic composition containing the mesenchymal stem cell obtained by the method of the present invention as described above as an effective ingredient can be prepared.

The mesenchymal stem cell obtained by the method of the present invention and the composition containing the cell are useful for treatment of diseases such as bone disease, chondropathy, cardiac disease, spinal cord injury, and graft-versus-host disease. Pharmaceutical composition for treatment of various diseases can be produced by using the mesenchymal stem cell obtained by the method of the present invention.

When the mesenchymal stem cell obtained by the method of the present invention is formulated into a pharmaceutical composition, the cell may be mixed with a pharmaceutically acceptable carrier by a conventional method to be formulated into a suitable dosage form for administration to individuals. Examples of the carrier include saline, and distilled water for injection which has become isotonic by addition of glucose or other adjuvants (for example, D-sorbitol, D-mannitol, sodium chloride, etc.). The pharmaceutical composition may further comprise a buffer (for example, phosphate buffer, sodium acetate buffer), a soothing agent (for example, benzalkonium chloride, procaine hydrochloride, etc.), a stabilizer (for example, human serum albumin, polyethylene glycol, etc.), a preservative, an antioxidant, and the like.

### (4) Production method of cell belonging to mesenchyme of the present invention

Various cells that are known to appear by differentiation from mesenchymal stem cells, that is, cells belonging to mesenchyme can be obtained by culturing the mesenchymal stem cell obtained by the method of the present invention in a medium containing a differentiation inducer that induces differentiation of a mesenchymal stem cell into another cell.

Examples of the cell belonging to mesenchyme obtained by the method of the present invention include, but not limited to, adipocyte (brown adipocyte, white adipocyte), chondrocyte, osteoblast, and muscular cell. For various cells belonging to mesenchyme, a method for inducing differentiation from mesenchymal stem cells is known. For example, dexamethasone, insulin, 3-isobutyl-1-methylxanthine, etc. for adipocytes; dexamethasone, ascorbic acid or ascorbic acid-2-phosphate, β-glycerophosphoric acid, etc. for bone cells; hydrocortisone, ascorbic acid, β-glycerophosphoric acid, etc. for osteoblasts; insulin, ascorbic acid-2-phosphate, hydrocortisone, etc. for chondrocytes are known as the differentiation inducers for the respective cells. The desired cells can be obtained by contacting the mesenchymal stem cells with these differentiation inducers.

Although the medium for differentiation induction as described above can be prepared as appropriate, a known medium or a commercially available medium may be used as it is or after alteration. Examples of a commercially available medium for differentiation induction include hMSC differentiation BulletKits-adipogenic medium (manufactured by LONZA) for differentiation to adipocytes, hMSC differentiation BulletKits-osteogenic medium (manufactured by LONZA) for differentiation to osteoblasts, and hMSC differentiation BulletKits-chondrogenic medium (manufactured by LONZA) for differentiation to chondrocytes.

The cell belonging to mesenchyme obtained by the above-mentioned method can be also formulated into a pharmaceutical composition. In such a case, a suitable composition or form is selected depending on the purposes or administration methods. These cells belonging to mesenchyme can be also used in screening of pharmaceutical candidate compounds or evaluation of safety of compounds.

### EXAMPLES

Hereinafter, the present invention will be further explained in detail by means of Examples to which the present invention is not limited.

### Example 1: Isolation and culture of adipose-derived stem cell

### (1) Separation of autologous plasma

Blood was withdrawn from a human healthy donor from whom informed consent was obtained via a blood collecting syringe. The blood was centrifuged at 500 x g for 20 minutes, and plasma was collected. The collected plasma was inactivated at 56°C for 30 minutes, cooled at 4°C for 30 minutes, and then centrifuged at 800 x g for 30 minutes. A supernatant was used as an inactivated autologous plasma (hereinafter, referred to as "autologous plasma").

### (2) Separation of stromal vascular fraction (SVF)

An adipose tissue was taken from the human healthy donor described in Example 1-(1), and stored in a saline. In a safety cabinet, the adipose tissue was put in a 10 cm culture dish and then cut into cubes 1-2 mm on a side by using sterile scissors. A new 10 cm culture dish was put on a scale and 15 g of the cut adipose tissue was weighed. To the adipose tissue was added 2 mg/mL of a Collagenase Type I/HBBSS solution in an amount of 3 mL per 1 g of the adipose tissue to suspend the adipose tissue therein. The suspension was kept 37°C for an hour in a constant-temperature bath while it was shaken at 100 rpm/minute. A centrifuge tube was put in the safety cabinet, and the suspension was filtered via 100 um mesh and then collected in the centrifuge tube. The suspension was centrifuged at 25°C and 200 x g for 10 minutes. A supernatant was carefully removed by using an electric pipettor. To a precipitate was added 10-20 mL of ACK lysing Buffer (manufactured by LONZA), and suspended. The suspension was left to stand at room temperature for 5 minutes, and then centrifuged at 25°C and 400 x g for 5 minutes. A supernatant was carefully removed from the centrifuged sample by using an electric pipettor to obtain a precipitate. To the precipitate was added a suitable amount of PBS and suspended to obtain a stromal vascular fraction.

### (3) Coating with RetroNectin (RN)

Culture instruments to be used in the following experiments were coated with RN. Specifically, 5 mL of a phosphate buffered saline (PBS) containing RetroNectin (registered trademark) (manufactured by TAKARA BIO INC.) (final concentration: 20 ug/mL) was added to a T-25 flask (manufactured by Corning) . As a control, a flask to which PBS not containing RetroNectin (referred to as "RN") was added was prepared.

These culture instruments were incubated at room temperature for an hour, and stored at 4°C until use. Just before use, the PBS was removed from the culture instruments by suction. The culture instruments were washed with PBS one time and then each cell culture medium one time, and then provided for cell culture. Hereinafter, the flask coated with RN is referred to as an RN flask.

### (3) Culture of stromal vascular fraction

A sample was obtained from the stromal vascular fraction obtained by Example 1-(2), and the total nucleated cell concentration was measured by Nucleo Counter (manufactured by Chemometec). Then, a cell suspension was prepared by adding a culture medium (a basic medium and an additive shown in Table 1) to the sample so that the total nucleated cell concentration became 1 × 10⁵ total nucleated cell number/mL. Then, 10 mL of the cell suspension was added to the RN flask or the control flask which was prepared by Example 1-(3). Cell culture was started at 37°C and under 5% CO₂ (Culture 0 day). One day after the start of culture, the medium was exchanged for a fresh medium, and thereafter the medium exchange was performed every 3-4 days until the cell reached 80-90% confluency.

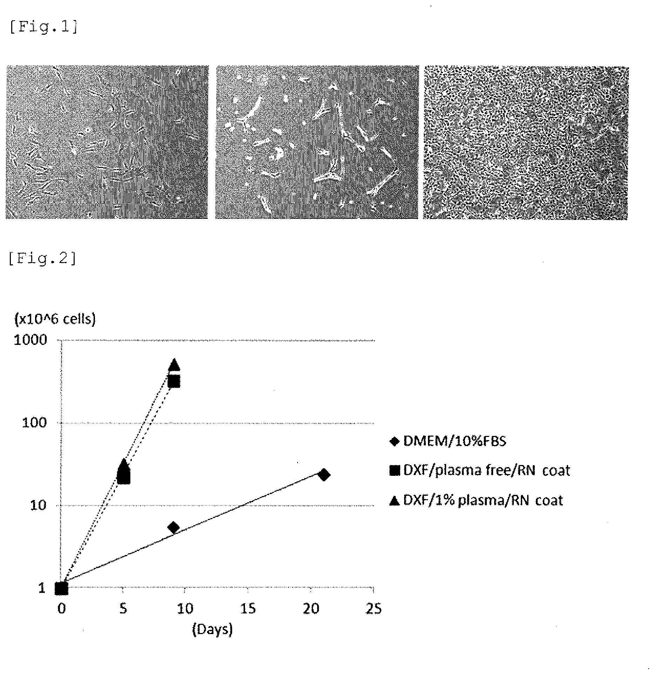

Basic media and additives used for the cell culture, the presence or absence of RN coating, the culture days spent until 80-90% confluency was reached, and the number of cells collected are shown in Table 1. A microscope photograph on the 8th day after the start of culture is shown in Figure 1.

**[Table 1]**

| Con dit ion | Basic medium (manufacturer) | Additive | RN | Number of cells collected (Culture days) |
|---|---|---|---|---|
| A | DMEM (SIGMA) | 10%FBS | No | 2.1 x 10⁵ (day 13) |
| B | DEF-CS500 XF (Cellartis) | 5%Autologous plasma | No | Not reach confluency |
| C | DEF-CS500 XF (Cellartis) | 5%Autologous plasma | Yes | 6.5 x 10⁵ (day 8) |

As can be seen from Table 1 and Figure 1, when DEF-CS500 XF to which 5% autologous plasma was added (Condition B) was used in place of DMEM to which 10% FBS was added (Condition A, Conventional method), the cell never reached the confluency. On the other hand, when the cell was cultured in DEF-CS500 XF to which 5% autologous plasma was added in the RN flask (Condition C), the cell growth rate greatly exceeded that in the conventional method (Condition A), and more cells were obtained in a shorter time.

Since the number of cells at the start of culture (1 × 10⁶) contained all cells in the stromal vascular fraction and the majority of the cells were excluded by the medium exchange on the 1^{st} day after the start of culture, the number of cells collected was smaller than 1 × 10⁶. The reason why the cell number at 80-90% confluency was largely different between Condition A and Condition C is that the size and shape of the cells were different.

### Example 2: Expansion of adipose-derived stem cell

### (1) Culture of stromal vascular fraction

In the same manner as Example 1-(4), the stromal vascular fraction obtained by Example 1-(2) was suspended in a culture medium so that the total nucleated cell concentration became 1 × 10⁵ total nucleated cell number/mL. Then, 10 mL of the cell suspension was added to the RN flask or the control flask which was prepared by Example 1-(3). Cell culture was started at 37°C and under 5% CO₂ (Culture 0 day). One day after the start of culture, the medium was exchanged for a fresh medium, and thereafter the medium exchange was performed every 3-4 days until the cell reached 80-90% confluency. Basic media and additives used for the cell culture, and the presence or absence of RN coating are shown in Table 2.

**[Table 2]**

| Con dit ion | Basic medium (manufacturer) | Additive | RN |
|---|---|---|---|
| D | DMEM | 10%FBS | No |
| | (SIGMA) | | |
| E | DXF | None | Yes |
| | (PromoCell) | | |
| F | DXF | 1%Autologous plasma | Yes |
| | (PromoCell) | | |

### (2) Expansion culture

When 80-90% confluency was reached, the cells were collected. A cell suspension was prepared by adding a culture medium (a basic medium and an additive shown in Table 2) to the cells so that the cell density became 1 × 10⁵ cells/mL. Then, 10 mL of the cell suspension was added to the RN flask or the control flask which was prepared by Example 1-(3). Expansion culture was started at 37°C and under 5% CO₂ (P0).

One day after the start of culture, the medium was exchanged for a fresh medium, and thereafter the medium exchange was performed every 3-4 days until the cell reached 70-95% confluency. At the time that 70-95% confluency was reached, the cells were collected (P1) and stained with trypan blue to measure the total nucleated cell concentration. Then, a cell suspension was prepared by adding a culture medium (a basic medium and an additive shown in Table 2) to the cells again so that the cell density became 1 × 10⁵ cells/mL. The cell suspension was added to a new RN flask or control flask and continued to be expanded at 37°C and under 5% CO₂. At the time that 80-90% confluency was reached, the cells were collected (P2) and stained with trypan blue to measure the total nucleated cell concentration. The culture days and the number of cells collected are shown in Table 3. Graphs of these results is shown in Figure 2.

**[Table 3]**

| Condition | P0 | P1 | P2 |
|---|---|---|---|
| D | 1.0 × 10⁶ | 5.4 × 10⁶ | 24 × 10⁶ |
| | (day 0) | (day 9) | (day 21) |
| E | | 22 × 10⁶ | 330 × 10⁶ |
| | | (day 5) | (day 9) |
| F | | 32 × 10⁶ | 530 × 10⁶ |
| | | (day 5) | (day 9) |

As can be seen from Table 3 and Figure 2, when the cell was cultured in DXF in the RN flask (Condition E and Condition F), more cells were obtained in a shorter time as compared with the cell cultured in DMEM to which 10% FBS was added (Condition D, Conventional method). Even in the case where the medium did not contain autologous plasma (Condition E), a cell growth rate greatly exceeded that in the conventional method (Condition D).

### Example 3: Analysis of cell surface marker

The P2 cell population obtained by Example 2-(2) was continued to be expanded under each culture condition while the cell was subcultured at the time that 80-90% confluency was reached. On about 20^{th} day after the start of expansion culture, the cells were collected and washed with PBS containing 0.1% bovine serum albumin (BSA, manufactured by SIGMA) (hereinafter, referred to as 0.1%BSA/PBS). The cell population was suspended in 0.1%BSA/PBS. To the suspension, each of antibody reaction solutions containing a PE-Cy7 labelled mouse anti-human CD73 antibody (manufacture by Becton, Dickinson and Company), an APC labelled mouse anti-human CD90 antibody (manufacture by Becton, Dickinson and Company), an APC labelled mouse anti-human CD105 antibody (manufacture by Becton, Dickinson and Company), a PE labelled mouse anti-human CD34 antibody (manufacture by Becton, Dickinson and Company), an FITC labelled mouse anti-human CD45 antibody (manufacture by Becton, Dickinson and Company), a PE-Cy7 labelled mouse anti-human HLA-DR antibody (manufacture by Becton, Dickinson and Company), an FITC labelled mouse anti-human CD11b antibody (manufacture by Becton, Dickinson and Company), and a PE labelled mouse anti-human CD14 antibody (manufacture by Becton, Dickinson and Company) was added, and the suspension was left to stand at room temperature for 10 minutes. Then, the cell population was washed with 0.1%BSA/PBS two times, and suspended in 0.1%BSA/PBS again. The cell population was subjected to flow cytometry (FACS CantoII, manufacture by Becton, Dickinson and Company) to calculate a rate of each cell surface marker contained in the cell population. Herein, CD73, CD90 and CD105 are cell surface markers for a mesenchymal stem cell (positive markers), and CD34, CD45, HLA-DR, CD11b and CD14 are negative markers which are not expressed on a mesenchymal stem cell. Results are shown in Table 4.

**[Table 4]**

| | Condition D P3 (Day 21) | Condition E P5 (Day 20) | Condition F P5 (Day 20) |
|---|---|---|---|
| CD73 | 99.9% | 99.6% | 99.4% |
| CD90 | 96.1% | 96.2% | 97.1% |
| CD105 | 99.8% | 100% | 100% |
| CD34 | 2.2% | less than a detection limit | less than a detection limit |
| CD45 | 2.4% | less than a detection limit | less than a detection limit |
| HLA-DR | 1.0% | less than a detection limit | less than a detection limit |
| CD11b | 2.9% | less than a detection limit | less than a detection limit |
| CD14 | 2.2% | less than a detection limit | less than a detection limit |

In Table, P3 means the cell population at the time that 80-90% confluency was reached after subculture of P2. P5 means the cell population at the time that 80-90% confluency was reached after two subcultures of P3.

As can be seen from Table 4, expression rates of the positive markers for a mesenchymal stem cell were 95% or more under all conditions. In contrast, the negative markers for a mesenchymal stem cell expressed a little in the conventional method (Condition D), whereas in the case where the cells were cultured in DXF in the RN flask (Condition E and Condition F), expression rates of all the negative markers was below the detection limit. In other words, it was found that a cell population containing a high proportion of mesenchymal stem cells can be efficiently obtained by cell culture in DXF in the RN flask as compared with the conventional method.

### Example 4: Differentiation into cell belonging to mesenchyme

The mesenchymal stem cells obtained by Example 3 (Condition E P5) were differentiated into various cells.

For differentiation into adipocytes, the mesenchymal stem cells (Condition E P5) were cultured in an hMSC differentiation BulletKits-adipogenic medium (manufactured by LONZA) according to an instruction. After removal of the medium, 4% paraformaldehyde (manufactured by SIGMA) was added to the cells, and left to stand at room temperature for 10 minutes to fix the cells. After the cells were washed with 60% isopropanol (manufactured by SIGMA), Oil-Red O was added to the cells, and incubated at room temperature for 15 minutes to visualize liquid drops of lipid.

For differentiation into osteoblasts, the mesenchymal stem cells (Condition E P5) were cultured in an hMSC differentiation BulletKits-osteogenic medium (manufactured by LONZA) according to an instruction. After removal of the medium, 4% paraformaldehyde (manufactured by SIGMA) was added to the cells, and left to stand at room temperature for 10 minutes to fix the cells. After the cells were washed with distilled water, an Alizarin Red staining solution was added to the cells, and incubated at room temperature for 45 minutes to visualize calcium deposition.

For differentiation into chondrocytes, the mesenchymal stem cells (Condition E P5) were cultured in an hMSC differentiation BulletKits-chondrogenic medium (manufactured by LONZA) according to an instruction. After removal of the medium, 4% paraformaldehyde (manufactured by SIGMA) was added to the cells, and left to stand at room temperature for 60 minutes to fix the cells. After the cells were washed with distilled water, an Alcian Blue staining solution was added to the cells, and incubated at room temperature overnight to visualize proteoglycan which is the main component of a cellular matrix forming cartilage.

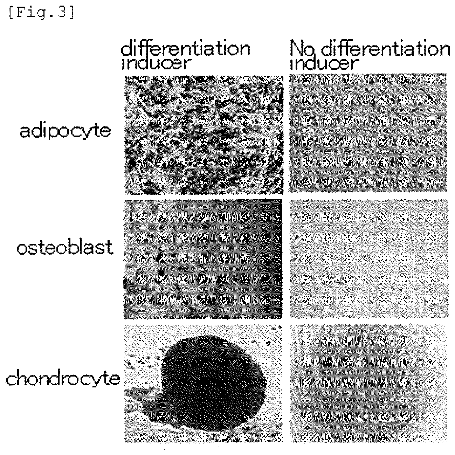

Results are shown in Figure 3. As can be seen from Figure 3, only when the cells were cultured in each differentiation induction medium, the cells were stained. These results show that the mesenchymal stem cells obtained by the method of the present invention will be differentiated into various cells known to appear by differentiation from mesenchymal stem cells, that is, cells belonging to mesenchyme, by culturing the mesenchymal stem cells in a medium containing a differentiation inducer.

### Industrial Applicability

According to the present invention, a method of producing a large number of mesenchymal stem cells in a short time is provided. The method of producing a mesenchymal stem cell of the present invention is particularly useful in regenerative medicine.

### Sequence Listing Free Text

SEQ ID NO:1; Partial region of fibronectin named III-8
SEQ ID NO:2; Partial region of fibronectin named III-9
SEQ ID NO:3; Partial region of fibronectin named III-10
SEQ ID NO:4; Partial region of fibronectin named III-11
SEQ ID NO:5; Partial region of fibronectin named III-12
SEQ ID NO:6; Partial region of fibronectin named III-13
SEQ ID NO:7; Partial region of fibronectin named III148
SEQ ID NO:8; Partial region of fibronectin named CS-1
SEQ ID NO:9; Fibronectin fragment named RetroNectin (CH-296)

## Claims

1. A method of producing a mesenchymal stem cell, the method comprising a step of culturing a cell population containing a mesenchymal stem cell in the presence of a fibronectin fragment using a medium containing plasma, wherein the cell population containing a mesenchymal stem cell is a cell population directly separated from adipose tissue.

2. The method according to claim 1, wherein the fibronectin fragment is a fragment having at least one domain selected from the group consisting of a cell-adhesion domain and a heparin-binding domain.

3. The method according to claim 1, wherein the culturing step in the presence of a fibronectin fragment is a step of culturing the cell population in contact with a solid phase coated with the fibronectin fragment.

4. The method according to claim 1, wherein the culturing step is a step of culturing the cell population in a xeno-free medium.

5. The method according to claim 4, wherein the xeno-free medium is a serum-free medium or a medium containing an allogeneic serum.

6. The method according to claim 1, wherein the cell population is a cell population derived from a human.

7. A method of producing a cell belonging to mesenchyme, the method comprising a step of obtaining a mesenchymal stem cell by the method according to any one of claims 1-6 and a step of differentiating the mesenchymal stem cell.

8. The method according to claim 7, wherein the differentiation step is a culturing step in a medium containing a differentiation inducer that induces differentiation of a mesenchymal stem cell into another cell.

## Patentansprüche

1. Verfahren zum Herstellen einer mesenchymalen Stammzelle, wobei das Verfahren einen Schritt eines Kultivierens einer Zellpopulation, die eine mesenchymale Stammzelle enthält, in der Gegenwart eines Fibronectin-Fragments unter Verwendung eines Mediums, das Plasma enthält, umfasst, wobei die Zellpopulation, die eine mesenchymale Stammzelle enthält, eine Zellpopulation, die direkt aus Fettgewebe separiert wurde, ist.

2. Verfahren gemäß Anspruch 1, wobei das Fibronectin-Fragment ein Fragment, das mindestens eine Domäne aufweist, die aus der Gruppe ausgewählt ist, die aus einer Zelladhäsionsdomäne und einer Heparinbindungsdomäne besteht, ist.

3. Verfahren gemäß Anspruch 1, wobei der Kultivierungsschritt in der Gegenwart eines Fibronectin-Fragments ein Schritt eines Kultivierens der Zellpopulation in Kontakt mit einer Festphase, die mit dem Fibronectin-Fragment beschichtet ist, ist.

4. Verfahren gemäß Anspruch 1, wobei der Kultivierungsschritt ein Schritt eines Kultivierens der Zellpopulation in einem xenofreiem Medium ist.

5. Verfahren gemäß Anspruch 4, wobei das xenofreie Medium ein serumfreies Medium oder ein Medium, das ein allogenes Serum enthält, ist.

6. Verfahren gemäß Anspruch 1, wobei die Zellpopulation eine Zellpopulation, die von einem Menschen abstammt, ist.

7. Verfahren zum Herstellen einer Zelle, die zum Mesenchym gehört, wobei das Verfahren einen Schritt eines Erhaltens einer mesenchymalen Stammzelle durch das Verfahren gemäß einem jeglichen der Ansprüche 1-6 und einen Schritt eines Differenzierens der mesenchymalen Stammzelle umfasst.

8. Verfahren gemäß Anspruch 7, wobei der Differenzierungsschritt ein Kultivierungsschritt in einem Medium, das ein Differenzierungsinduktionsmittel enthält, das eine Differenzierung einer mesenchymalen Stammzelle in eine andere Zelle induziert, ist.

## Revendications

1. Procédé de production d'une cellule souche mésenchymateuse, le procédé comprenant une étape de culture d'une population cellulaire contenant une cellule souche mésenchymateuse en présence d'un fragment de fibronectine en utilisant un milieu contenant du plasma, dans lequel la population cellulaire contenant une cellule souche mésenchymateuse est une population cellulaire directement séparée du tissu adipeux.

2. Procédé selon la revendication 1, dans lequel le fragment de fibronectine est un fragment ayant au moins un domaine choisi dans le groupe constitué par un domaine d'adhésion cellulaire et un domaine de liaison à l'héparine.

3. Procédé selon la revendication 1, dans lequel l'étape de culture en présence d'un fragment de fibronectine est une étape de culture de la population cellulaire en contact avec une phase solide revêtue du fragment de fibronectine.

4. Procédé selon la revendication 1, dans lequel l'étape de culture est une étape de culture de la population cellulaire dans un milieu exempt de xéno-contaminants.

5. Procédé selon la revendication 4, dans lequel le milieu exempt de xéno-contaminants est un milieu sans sérum ou un milieu contenant un sérum allogénique.

6. Procédé selon la revendication 1, dans lequel la population cellulaire est une population cellulaire dérivée d'un humain.

7. Procédé de production d'une cellule appartenant au mésenchyme, le procédé comprenant une étape d'obtention d'une cellule souche mésenchymateuse par le procédé selon l'une quelconque des revendications 1 à 6 et une étape de différenciation de la cellule souche mésenchymateuse.

8. Procédé selon la revendication 7, dans lequel l'étape de différenciation est une étape de culture dans un milieu contenant un inducteur de différenciation qui induit la différenciation d'une cellule souche mésenchymateuse en une autre cellule.
